# EUROPÄISCHE PATENTANMELDUNG

(11) **EP 1 481 728 A1**
(43) Veröffentlichungstag der Anmeldung: **01.12.2004**
(21) Anmeldenummer: 04015937.8
(22) Anmeldetag: 07.04.1999
(51) Int. Cl.: B01J 21/00, B01J 29/04, B01J 37/00, C07D 301/12

(54) **Verfahren zur Herstellung eines keramischen Formkörpers unter Verwendung eines Metalloxidsols, Formkörper und seine verwendung bei der Herstellung eines Alkenoxids**

(30) Priorität: 08.04.1998 DE 19815879; 22.12.1998 DE 19859561
(62) Teilanmeldung aus: 99914571.7
(71) Anmelder: BASF AKTIENGESELLSCHAFT, 67056 Ludwigshafen (DE)
(72) Erfinder: Grosch, Georg-Heinrich, Dr., 67098 Bad Dürkheim (DE); Müller, Ulrich, Dr., 67435 Neustadt (DE); Hesse, Michael, Dr., 67549 Worms (DE); Lockemann, Christian, Dr., 68163 Mannheim (DE)
(74) Vertreter: Isenbruck, Günter, Dr.

(57) **Zusammenfassung**

Die vorliegende Erfindung betrifft ein Verfahren zur Herstellung eines Formkörpers, umfassend mindestens ein poröses oxidisches Material und mindestens ein Metalloxid, sowie den Formkörper an sich und dessen Verwendung, wobei das Verfahren die folgenden Stufe (i) bis (iii) umfaßt:
(i) Vermischen des mindestens einen porösen oxidischen Materials mit mindestens einem Metalloxidsol, das einen niedrigen Gehalt an Alkali- und Erdalkalimetallionen aufweist, und/oder mindestens einem Metalloxid, das einen niedrigen Gehalt an Alkali- und Erdalkalimetallionen aufweist, durch Versprühen einer Suspension, enthaltend das mindestens eine poröse oxidische Material und das Metalloxidsol und/oder Metalloxid,
(ii) Verdichten des Gemisches aus Stufe (i) unter Zusatz von Metalloxidsol,
(iii) Verformen der Masse aus Stufe (ii),
wobei das in (i) oder (i) und (ii) eingesetzte Metalloxidsol einen Gehalt an Alkali- und Erdalkalimetallionen von weniger als 10 ppm und das in (i) eingesetzte Metalloxid einen Gehalt an Alkali- und Erdalkalimetallionen von weniger als 200 ppm aufweisen.

## Beschreibung

Die vorliegende Erfindung betrifft ein Verfahren zur Herstellung eines Formkörpers, der mindestens ein poröses oxidisches Material und mindestens ein Metalloxid enthält, den Formkörper an sich sowie dessen Verwendung als Katalysator bei der Umsetzung von organischen Verbindungen, insbesondere zur Epoxidation von organischen Verbindungen, die mindestens eine C-C-Doppelbindung aufweisen.

Formkörper, die poröse oxidische Materialen enthalten, werden in zahlreichen chemischen Verfahren eingesetzt. Notwendig dazu ist ein Herstellungsverfahren, das es erlaubt, kostengünstig großtechnisch relevante Mengen an Formkörpern herzustellen.

In der Regel wird zur Herstellung von Formkörpern das poröse oxidische Material mit einem Binder, einer organischen viskositätssteigernden Substanz und einer Flüssigkeit zum Anteigen der Masse versetzt und in einem Kneter oder Koller verdichtet. Anschließend wird die erhaltene Masse mittels Strangpresse oder Extruder verformt, und die erhaltenen Formkörper werden getrocknet und calciniert.

Um Formkörper herzustellen, die auch zur Herstellung sehr reaktiver Produkte geeignet sind, ist es notwendig, chemisch inerte Binder zu verwenden, die eine Weiterreaktion dieser Produkte verhindern.

Geeignete Binder stellen eine Reihe von Metalloxiden dar. Beispielsweise seien Oxide des Siliciums, des Aluminiums, des Titans oder des Zirkons genannt. Siliciumdioxid als Binder ist beispielsweise in den Druckschriften US 5,500,199 und US 4,859,785 offenbart.
Bei derartigen Bindern sollte der Gehalt an (Erd)alkalimetallionen möglichst niedrig sein, weshalb eine Verwendung von (erd)alkalimetallarmen bzw. (erd)alkalimetallfreien Binderquellen notwendig ist.

Zur Herstellung der oben genannten Metalloxid-Binder kann man als Edukte entsprechende Metalloxidsole verwenden. Bei der Herstellung von beispielsweise der erwähnten (erd)alkalimetallarmen bzw. (erd)alkalimetallfreien Siliciumdioxid-Binder dient demgemäß (erd)alkalimetallarmes bzw. (erd)alkalimetallfreies Kieselsol als Binderquelle.

Bei der Herstellung von Kieselsolen kann man von Alkalisilicaten ausgehen, was jedoch in der Regel zu unerwünscht hohen Gehalten an Alkalimetallionen im Kieselsol führt. Die Herstellung solcher Kieselsole ist beispielsweise in "Ullmann's Encyclopedia of Industrial Chemistry", Band A23 (1993), S. 614-629 beschrieben.

Die JP-A-07 048 117 offenbart die Kieselsolherstellung durch Hydrolyse von Alkoxysilanen unter Einwirkung von Ammoniak in Gegenwart eines großen Überschusses von Alkohol, wobei die erhaltenen Kieselsole einen Gehalt von bis zu 10 Gew.-% an Siliciumdioxid haben.

Die JP-A-05 085 714 beschreibt die saure Zersetzung von Alkoxysilanen, ebenfalls in alkoholischem Medium. Dabei werden Kieselsole mit Siliciumdioxidgehalten von 1 bis 10 Gew.-% erhalten.
Ein Nachteil der in den beiden letzten Druckschriften offenbarten Verfahren zur Kieselsolherstellung ist der niedrige erreichbare Siliciumdioxidgehalt der Kieselsole, der das Verfahren unwirtschaftlich macht, da sowohl bei der Solherstellung als auch bei einer Weiterverarbeitung Anlagenkapazität durch überflüssige Wassermasse vergeudet wird.

Eine Aufgabe der vorliegenden Erfindung ist es demgemäß, ein großtechnisch einsetzbares Verfahren zur Herstellung von Formkörpern bereitzustellen, die einen niedrigen Gehalt an (Erd)alkalimetallionen aufweisen und die als Katalysatoren, vorzugsweise im Festbett, verwendet werden können.

Überraschend wurde gefunden, daß solche Formkörper erhalten werden können, wenn in einer Stufe des Verfahrens poröses oxidisches Material mit Metalloxidsol und/oder Metalloxid vermischt wird, wobei das Metalloxidsol und das Metalloxid jeweils einen niedrigen Gehalt an (Erd)alkalimetallionen aufweisen.

Demgemäß betrifft die vorliegende Erfindung ein Verfahren zur Herstellung eines Formkörpers, umfassend mindestens ein poröses oxidisches Material und mindestens ein Metalloxid, das die folgende Stufe (i) umfaßt:
(i) Vermischen des mindestens einen porösen oxidischen Materials mit mindestens einem Metalloxidsol, das einen niedrigen Gehalt an Alkali- und Erdalkalimetallionen aufweist, und/oder mindestens einem Metalloxid, das einen niedrigen Gehalt an Alkali- und Erdalkalimetallionen aufweist.

Ebenso betrifft die vorliegende Erfindung einen Formkörper, herstellbar durch vorstehend beschriebenes Verfahren, wobei dieser Formkörper einen Gehalt an (Erd)alkalimetallionen von bevorzugt weniger als 700 ppm, besonders bevorzugt weniger als 600 ppm und insbesondere weniger als 500 ppm aufweist.
In einer bevorzugten Ausführungsform des erfindungsgemäßen Verfahrens wird das Metalloxidsol durch Hydrolyse mindestens eines Metallsäureesters hergestellt. Daher betrifft die vorliegende Erfindung auch ein wie oben beschriebenes Verfahren, das dadurch gekennzeichnet ist, daß das Metalloxidsol hergestellt wird durch Hydrolyse mindestens eines Metallsäureesters.
Die zur Hydrolyse anstehenden Metallsäureester können vor der Hydrolyse gereinigt werden. Dabei sind alle geeigneten Verfahren denkbar. Bevorzugt werden die Metallsäureester vor der Hydrolyse einer Destillation unterzogen.

Hinsichtlich der Hydrolyse des Metallsäureesters kommen prinzipiell alle möglichen Verfahren in Betracht. Bevorzugt wird im erfindungsgemäßen Verfahren die Hydrolyse jedoch im wäßrigen Medium durchgeführt. Dies bietet den Vorteil, daß im Vergleich zu aus der Literatur, beispielsweise der JP 07,048,117 oder der JP 05,085,714, bekannten Hydrolysen, in denen bei Alkoholüberschuß gearbeitet wird, wesentlich weniger Alkohol abdestilliert werden muß.

Katalysiert werden kann die Hydrolyse durch Zugabe basischer oder saurer Substanzen. Bevorzugt werden dabei basische oder saure Substanzen, die sich durch Calcinieren rückstandsfrei entfernen lassen. Vor allem werden Substanzen, ausgewählt aus der Gruppe Ammoniak, Alkylamine, Alkanolamine, Arylamine, Carbonsäuren, Salpetersäure und Salzsäure, verwendet. Insbesondere werden Ammoniak, Alkylamine, Alkanolamine und Carbonsäuren verwendet.

Bevorzugt wird im erfindungsgemäßen Verfahren als Metallsäureester Orthokieselsäureester verwendet.

Die Hydrolyse der Metallsäureester erfolgt im erfindungsgemäßen Verfahren bei Temperaturen von 20 bis 100 °C, bevorzugt von 60 bis 95 °C sowie bei pH-Werten von 4 bis 10, bevorzugt von 5 bis 9, besonders bevorzugt von 7 bis 9.

Das Molverhältnis katalytisch wirkende Substanz / Metallsäureester beträgt im allgemeinen 0,0001 bis 0,11, bevorzugt 0,0002 bis 0,01 und insbesondere 0,0005 bis 0,008.

Im erfindungsgemäßen Verfahren werden aus der Hydrolyse Metalloxidsole, vorzugsweise Kieselsole erhalten, die einen Gehalt an (Erd)alkalimetallionen von weniger als 800 ppm, bevorzugt weniger als 600 ppm, weiter bevorzugt weniger als 400 ppm, weiter bevorzugt weniger als 200 ppm, weiter bevorzugt weniger als 100 ppm, besonders bevorzugt weniger als 50 ppm, weiter besonders bevorzugt weniger als 10 ppm, insbesondere weniger als 5 ppm aufweisen.

Demgemäß betrifft die vorliegende Erfindung ein Metalloxidsol mit einem Gehalt an (Erd)alkalimetallionen von weniger als 800 ppm, herstellbar durch Hydrolyse mindestens eines Metallsäureesters.

Der Gehalt der erfindungsgemäß hergestellten Metalloxidsole an Metalloxid beträgt im allgemeinen bis zu 50 Gew.-%, bevorzugt 10 bis 40 Gew.-%.

Der bei der Hydrolyse entstehende Alkohol wird im erfindungsgemäßen Verfahren in der Regel abdestilliert. Geringe Mengen an Alkohol können jedoch im Metalloxidsol verbleiben, solange sie in den weiteren Schritten des erfindungsgemäßen Verfahrens keine störenden Auswirkungen zeigen.

Vorteilhaft für den großtechnischen Einsatz der erfindungsgemäß hergestellten Metalloxidsole ist deren Eigenschaft, keine Tendenzen zur Gelbildung zu zeigen. Spezielle Vorsichtmaßnahmen zur Verhinderung der Gelbildung sind damit überflüssig. Die Lagerfähigkeit der erfindungsgemäß hergestellten Metalloxidsole beträgt mehrere Wochen, wodurch die zeitliche Koordination mit weiteren Verfahrensschritten unproblematisch wird.

Erfindungsgemäß wird im Verfahren ein Gemisch, umfassend mindestens ein poröses oxidisches Material und mindestens ein Metalloxid, hergestellt, wobei als Metalloxidquelle ein Metalloxidsol verwendet wird, das wie oben beschrieben hergestellt wird.

Prinzipiell bestehen keine Einschränkungen bezüglich des Verfahrens zur Herstellung des Gemisches. Bevorzugt wird im erfindungsgemäßen Verfahren jedoch eine Suspension, enthaltend mindestens ein poröses oxidisches Material und Metalloxidsol, versprüht.

Dabei bestehen hinsichtlich des Gehaltes der Suspension an porösem oxidischem Material keine Einschränkungen, solange die Verarbeitbarkeit der Suspension beim Herstellen und Versprühen gewährleistet ist. Bevorzugt wird das Gewichtverhältnis von porösem oxidischem Material zum Metalloxid des Metalloxidsols von 10 bis 0,1 gewählt, besonders bevorzugt von 8 bis 1.

Die Hauptbestandteile der Suspension sind im allgemeinen poröses oxidisches Material, Metalloxidsol und Wasser. Ferner kann die Suspension noch Restspuren organischer Verbindungen enthalten. Diese können beispielsweise aus der Herstellung des porösen oxidischen Materials stammen. Ebenso sind Alkohole denkbar, die aus der Hydrolyse von Metallsäureester entstehen oder Substanzen, die wie oben beschrieben zur Förderung der Hydrolyse von Metallsäureester zugesetzt werden.

In Abhängigkeit davon, welche Feuchtigkeit das Gemisch zur weiteren Verarbeitung aufweisen soll, kann sich eine Trocknung anschließen. Dabei können alle denkbaren Verfahren angewendet werden. Bevorzugt erfolgt die Trocknung des Gemisches gleichzeitig mit dem Versprühen in einem Sprühtrocknungsvorgang. Bevorzugt werden die Sprühtrockner mit Inertgasen, besonders bevorzugt mit Stickstoff oder Argon betrieben.

Bezüglich der im erfindungsgemäßen Verfahren zur Herstellung der Formkörper verwendbaren porösen oxidischen Materialien existieren keine besonderen Beschränkungen, solange es möglich ist, ausgehend von diesen Materialien wie hierin beschriebene Formkörper herzustellen, und solange diese Materialien die notwendige katalytische Aktivität aufweisen.

Vorzugsweise ist das poröse oxidische Material ein Zeolith. Zeolithe sind bekanntermaßen kristalline Alumosilicate mit geordneten Kanal- und Käfigstrukturen, die Mikroporen aufweisen. Der Begriff "Mikroporen", wie er im Rahmen der vorliegenden Erfindung verwendet wird, entspricht der Definition in "Pure Appl. Chem." 57 (1985) S. 603-619, und bezeichnet Poren mit einem Porendurchmesser von kleiner 2 nm. Das Netzwerk solcher Zeolithe ist aufgebaut aus SiO₄- und AlO₄-Tetraedern, die über gemeinsame Sauerstoffbrücken verbunden sind. Eine Übersicht über die bekannten Strukturen findet sich beispielsweise bei W. M. Meier, D. H. Olson und Ch. Baerlocher in "Atlas of Zeolite Structure Types", Elsevier, 4. Auflage, London 1996.

Ferner existieren Zeolithe, die kein Aluminium enthalten und bei denen im Silicatgitter anstelle des Si(IV) teilweise Titan als Ti(IV) vorhanden ist. Die Titanzeolithe, insbesondere solche mit einer Kristallstruktur vom MFI-Typ, sowie Möglichkeiten zu ihrer Herstellung sind beschrieben beispielsweise in der EP-A 0 311 983 oder der EP-A 0 405 978. Außer Silicium und Titan können solche Materialien auch zusätzliche Elemente wie z.B. Aluminium, Zirkonium, Zinn, Eisen, Niob, Kobalt, Nickel, Gallium, Bor oder geringe Mengen an Fluor enthalten.

In den beschriebenen Zeolithen kann das Titan desselben teilweise oder vollständig durch Vanadium, Zirkonium, Chrom, Niob oder Eisen oder durch ein Gemisch aus zwei oder mehr davon ersetzt sein. Das molare Verhältnis von Titan und/oder Vanadium, Zirkonium, Chrom, Niob oder Eisen zur Summe aus Silicium und Titan und/oder Vanadium, Zirkonium, Chrom, Niob oder Eisen liegt in der Regel im Bereich von 0,01: 1 bis 0,1:1.

Titanzeolithe mit MFI-Struktur sind dafür bekannt, daß sie über ein bestimmtes Muster bei der Bestimmung ihrer Röntgenbeugungsaufnahmen sowie zusätzlich über eine Gerüstschwingungsbande im Infrarotbereich (IR) bei etwa 960 cm⁻¹ identifiziert werden können und sich damit von Alkalimetalltitanaten oder kristallinen und amorphen TiO₂-Phasen unterscheiden.

Vorzugsweise werden Titan-, Vanadium-, Chrom-, Niob-, Zirkonium-Zeolithe, weiter bevorzugt Titan-Zeolithe und insbesondere Titansilicalite verwendet.

Dabei sind im einzelnen Titan-, Vanadium-, Chrom-, Niob-, Zirkonium-Zeolithe mit Pentasil-Zeolith-Struktur, insbesondere die Typen mit röntgenografischer Zuordnung zur BEA-, MOR-, TON-, MTW-, FER-, MFI-, MEL-, CHA-, ERI-, RHO-, GIS-, BOG-, NON-, EMT-, HEU-, KFI-, FAU-, DDR-, MTT-, RUT-, RTH-, LTL-, MAZ-, GME-, NES-, OFF-, SGT-, EUO-, MFS-, MWW- oder MFI/MEL-Mischstruktur sowie ITQ-4 zu nennen. Zeolithe dieses Typs sind beispielsweise in der oben genannten Literaturstelle von Meier et al. beschrieben. Denkbar sind für den Einsatz im erfindungsgemäßen Verfahren weiterhin titanhaltige Zeolithe mit der Struktur des UTD-1, CIT-1 oder CIT-5. Als weitere titanhaltige Zeolithe sind solche mit der Struktur des ZSM-48 oder ZSM-12 zu nennen.

Derartige Zeolithe sind unter anderem in der US-A 5 430 000 und der WO 94/29408 beschrieben, deren diesbezüglicher Inhalt voll umfänglich in die vorliegende Anmeldung durch Bezugnahme aufgenommen wird. Als besonders bevorzugt sind für das erfindungsgemäße Verfahren Ti-Zeolithe mit MFI-, MELoder MFI/MEL-Mischstruktur anzusehen. Als weiter bevorzugt sind im einzelnen die Ti-enthaltenden Zeolith-Katalysatoren, die im allgemeinen als "TS-1", "TS-2", "TS-3" bezeichnet werden, sowie Ti-Zeolithe mit einer zu β-Zeolith isomorphen Gerüststruktur zu nennen.

Dementsprechend betrifft die vorliegende Erfindung ein Verfahren zur Herstellung eines Formkörpers, wie oben beschrieben, das dadurch gekennzeichnet ist, daß das poröse oxidische Material ein Zeolith ist.

Selbstverständlich können im erfindungsgemäßen Verfahren auch Gemische aus zwei oder mehr, insbesondere der oben genannten porösen oxidischen Materialien verwendet werden.

Üblicherweise stellt man die genannten Titan-, Zirkonium-, Chrom-, Niob-, Eisen- und Vanadiumzeolithe dadurch her, daß man eine wäßrige Mischung aus einer Metalloxidquelle, vorzugsweise einer SiO₂-Quelle, sowie aus einer Titan-, Zirkonium-, Chrom-, Niob-, Eisen- bzw. Vanadium-Quelle, wie z.B. Titandioxid bzw. einem entsprechenden Vanadiumoxid, Zirkoniumalkoholat, Chromoxid, Nioboxid oder Eisenoxid und einer stickstoffhaltigen organischen Base als Templat ("Schablonen-Verbindung"), wie z.B. Tetrapropylammoniumhydroxid, gegebenenfalls noch unter Hinzufügen basischer Verbindungen, in einem Druckbehälter unter erhöhter Temperatur im Zeitraum mehrerer Stunden oder einiger Tage umsetzt, wobei ein kristallines Produkt entsteht. Dieses wird abfiltriert, gewaschen, getrocknet und zur Entfernung der organischen Stickstoffbase bei erhöhter Temperatur gebrannt. In dem so erhaltenen Pulver liegt das Titan bzw. das Zirkonium, Chrom, Niob, Eisen und/oder Vanadium zumindest teilweise innerhalb des Zeolithgerüsts in wechselndem Anteil mit 4-, 5- oder 6-facher Koordination vor. Zur Verbesserung des katalytischen Verhaltens kann sich noch eine mehrmalige Waschbehandlung mit schwefelsaurer Wasserstoffperoxidlösung anschließen, worauf das Titan- bzw. Zirkonium-, Chrom-, Niob-, Eisen-, Vanadiumzeolith-Pulver erneut getrocknet und gebrannt werden muß. Das so hergestellte Titan- bzw. Zirkonium-, Chrom-, Niob-, Eisen-, oder Vanadiumzeolith-Pulver wird im erfindungsgemäßen Verfahren als Komponente der oben beschriebenen Suspension verwendet.

Insbesondere betrifft die vorliegende Erfindung daher ein Verfahren, wie oben beschrieben, in dem das mindestens eine poröse oxidische Material mit mindestens einem Metalloxidsol vermischt wird, wobei das mindestens eine poröse oxidische Material hergestellt wird in einem Verfahren, das eine oder mehrere der folgenden Stufen (a) bis (f) umfaßt:
(a) Herstellen einer vorzugsweise wäßrigen Mischung aus mindestens einer Metalloxidquelle, vorzugsweise einer SiO₂-Quelle, und einer weiteren Metallquelle, beispielsweise einer Titan-, Zirkonium-, Chrom-, Niob-, Eisen- bzw. Vanadium-Quelle,
(b) Kristallisation der Mischung aus (a) in einem Druckbehälter unter Zugabe mindestens einer Schablonenverbindung, gegebenenfalls unter Zugabe einer weiteren basischen Verbindung,
(c) Trocknung des in der aus (b) resultierenden Suspension enthaltenen kristallinen Produktes, vorzugsweise durch Sprühtrocknung,
(d) Calcinieren des getrockneten Produktes aus (c),
(e) Zerkleinern des calcinierten Produktes aus (d), beispielsweise durch Mahlen, zu Partikeln mit Partikelgrößen kleiner 500 µm, bevorzugt kleiner 300 µm, besonders bevorzugt kleiner 200 µm,
(f) gegebenenfalls mehrmalige Waschbehandlung des zerkleinerten Produktes aus (e) mit anschließender Trocknung und Calcinierung.

Auch bezüglich der Porenstruktur der porösen oxidischen Materialien existieren keine besonderen Beschränkungen, d.h. das Material kann Mikroporen, Mesoporen, Makroporen, Mikro- und Mesoporen, Mikro- und Makroporen, Meso- und Makroporen oder Mikro-, Meso- und Makroporen aufweisen, wobei die Definition der Begriffe "Mesoporen" und "Makroporen" ebenfalls derjenigen in oben erwähnter Literatur gemäß "Pure Appl. Chem." entspricht und Poren mit einem Durchmesser von > 2 nm bis ca. 50 nm bzw. > ungefähr 50 nm bezeichnet.

Vorzugsweise werden jedoch mikroporöse oxidische Materialien wie beispielsweise Titansilicalite verwendet.

In einer weiteren bevorzugten Ausführungsform des erfindungsgemäßen Verfahrens wird das mindestens eine poröse oxidische Material in Schritt (i) mit mindestens einem Metalloxid vermischt, das einen niedrigen Gehalt an Alkaliund Erdalkalimetallionen aufweist.

In dem Fall, daß das poröse oxidische Material mit zwei oder mehr Metalloxiden vermischt wird, ist es denkbar, das mindestens eine poröse oxidische Material zuerst mit einem Metalloxid zu vermischen und das erhaltene Gemisch mit einem weiteren Metalloxid zu vermischen. Falls gewünscht, kann dieses erhaltene Gemisch wiederum mit einem weiteren Metalloxid vermischt werden. Ebenso ist es möglich, das poröse oxidische Material mit einem Gemisch aus zwei oder mehr Metalloxiden zu vermischen.

Der (Erd)alkalimetallgehalt dieses Metalloxides oder des Gemisches aus zwei oder mehr Metalloxiden ist im allgemeinen kleiner als 800 ppm, bevorzugt kleiner 600 ppm, besonders bevorzugt kleiner 500 ppm und insbesondere bevorzugt kleiner 200 ppm.

Solche Metalloxide mit einem niedrigen Gehalt an Alkali- und Erdalkalimetallionen sind beispielsweise pyrogene Metalloxide, wobei als Beispiel für ein solches pyrogenes Metalloxid unter anderem pyrogene Kieselsäure genannt sei.

Selbstverständlich ist es auch möglich, konventionelle Metalloxide im erfindungsgemäßen Verfahren einzusetzen, unter der Maßgabe, daß deren Gehalt an Alkali- und Erdalkalimetallionen entsprechend, wie oben angegeben, niedrig ist.

Denkbar ist auch, bei einem oder mehreren konventionellen Metalloxiden, die einen höheren als den oben angegebenen Gehalt an Alkali- und Erdalkalimetallionen aufweisen, durch Waschen, Extraktion oder andere geeignete Maßnahmen, ebenso natürlich durch eine Kombination von zwei oder mehr geeigneter Maßnahmen, den Gehalt an Alkali- und Erdalkalimetallionen soweit abzusenken, daß die Metalloxide im erfindungsgemäßen Verfahren eingesetzt werden können.

Dabei kann es, je nach ergriffener Maßnahme zur Verringerung des Gehaltes an Alkali- und Erdalkalimetallionen, nötig sein, das eine oder die mehreren konventionellen Metalloxid(e) entsprechend nachzubehandeln. Wird beispielsweise der Gehalt an Alkali- und Erdalkalimetallionen eines konventionellen Metalloxids durch Waschen verringert, so ist es unter Umständen nötig, das konventionelle Metalloxid nach dem Waschen zu trocknen, bevor es mit dem mindestens einen porösen oxidischen Material vermischt wird.

Im Rahmen des erfindungsgemäßen Verfahrens ist es natürlich auch möglich, das Gemisch, das aus dem Vermischen des mindestens einen porösen oxidischen Materials mit dem Metalloxid resultiert, mit mindestens einem Metalloxidsol, das einen niedrigen Gehalt an Alkali- und Erdalkalimetallionen aufweist, zu vermischen. Bezüglich der Herstellung dieses Gemisches bestehen, wie bei der Herstellung des Gemisches aus porösem oxidischem Material und Metalloxidsol, wie oben beschrieben, prinzipiell keine Einschränkungen. Bevorzugt wird jedoch eine Suspension, umfassend das Gemisch aus dem mindestens einen porösen oxidischen Material und dem mindestens einen Metalloxid und das mindestens eine Metalloxidsol, versprüht. Hinsichtlich des Gehaltes dieser Suspension an porösem oxidischem Material bestehen keine Einschränkungen, solange, wie bereits oben beschrieben,die Verarbeitbarkeit der Suspension gewährleistet ist.

Weiterhin ist es im Rahmen des erfindungsgemäßen Verfahrens natürlich auch möglich, ein Gemisch, das aus der Vermischung von mindestens einem porösen oxidischen Material mit mindestens einem Metalloxidsol resultiert, mit mindestens einem Metalloxid, das einen niedrigen Gehalt an Alkali- und Erdalkalimetallionen aufweist, zu vermischen. Dabei kann sich die Vermischung mit dem mindestens einen Metalloxid direkt an die Herstellung des Gemisches aus dem mindestens einen porösen oxidischen Material und dem mindestens einen Metalloxidsol anschließen. Sollte, wie bereits oben beschrieben, nach der Herstellung des Gemisches aus dem mindestens einen porösen oxidischen Material und dem mindestens einen Metalloxidsol eine Trocknung erforderlich sein, ist es auch möglich, das Metalloxid nach der Trocknung mit dem getrockneten Gemisch zu vermischen.

Ebenso ist es im Rahmen des erfindungsgemäßen Verfahrens möglich, das mindestens eine poröse oxidische Material gleichzeitig mit mindestens einem Metalloxidsol und mindestens einem Metalloxid zu vermischen.

Das Gemisch, das nach einer der obenstehend beschriebenen Ausführungsformen der Erfindung erhalten wird, wird in einer weiteren Stufe des erfindungsgemäßen Verfahrens verdichtet. In diesem Verdichtungs- oder Verformungsschirtt kann dabei optional weiteres Metalloxid eingebracht werden, wobei als Metalloxidquelle Metalloxidsol dient, das wie oben beschrieben hergestellt wurde. Dieser Verarbeitungsschritt kann in allen dafür bekannten Apparaturen erfolgen, wobei jedoch Kneter, Koller oder Extruder bevorzugt sind. Besonders bevorzugt für den großtechnischen Einsatz des erfindungsgemäßen Verfahrens wird ein Koller verwendet.

Wird gemäß einer bereits oben beschriebenen Ausführungsform zuerst ein Gemisch aus mindestens einem porösen oxidischen Material und mindestens einem Metalloxid hergestellt, dieses Gemisch verdichtet und im Verdichtungsschritt zusätzlich Metalloxidsol, das einen niedrigen Gehalt an Alkali- und Erdalkalimetallionen aufweist, zugegeben, so werden in einer bevorzugten Ausführungsform der vorliegenden Erfindung 20 bis 80 Gew.-% poröses oxidisches Material, 10 bis 60 Gew.-% Metalloxid und 5 bis 30 Gew.-% Metalloxidsol verwendet. Besonders bevorzugt werden 40 bis 70 Gew.-% poröses oxidisches Material, 15 bis 30 Gew.-% Metalloxid und 10 bis 25 Gew.-% Metalloxidsol verwendet. Diese Gewichtsprozentangaben sind jeweils bezogen auf den letztlich hergestellten Formkörper, wie untenstehend beschrieben. Bevorzugt werden hierbei poröses oxidisches titanhaltiges Material und Kieselsol eingesetzt.

In einer weiteren Ausführungsform des erfindungsgemäßen Verfahrens erfolgt das Vermischen des mindestens einen porösen oxidischen Materials mit dem mindestens einen Metalloxid, das einen niedrigen Gehalt an Alkali- und Erdalkalimetallionen aufweist, während des Verdichtungsschritts. Ebenso ist es demgemäß möglich, im Verdichtungsschritt das mindestens eine poröse oxidische Material, das mindestens eine Metalloxid und zusätzlich mindestens ein Metalloxidsol zu vermischen.

In diesem Verformungsschritt können zusätzlich eine oder mehrere vikositätssteigemde Substanzen als Anteigungsmittel zugegeben werden, die unter anderem dazu dienen, die Stabilität des uncalcinierten Formkörpers, wie untenstehend beschrieben, zu erhöhen. Dafür können alle geeigneten, aus dem Stand der Technik bekannten Substanzen verwendet werden. Im erfindungsgemäßen Verfahren werden Wasser sowie Mischungen von Wasser mit einer oder mehreren organischen Substanzen, sofern diese mit Wasser mischbar sind, als Anteigungsmittel verwendet. Das Anteigungsmittel kann beim späteren Calcinieren des Formkörpers wieder entfernt werden.

Vorzugsweise werden organische, insbesondere hydrophile organische Polymere wie z.B. Cellulose, Cellulosederivate wie beispielsweise Methylcellulose, Ethylcellulose oder Hexylcellulose, Polyvinylpyrolidon, Ammonium(meth)acrylate, Tylose oder Gemische aus zwei oder mehr davon verwendet. Besonders bevorzugt wird Methylcellulose verwendet.

Als weitere Zusatzstoffe können Ammoniak, Amine oder aminartige Verbindungen wie z.B. Tetraalkylammoniumverbindungen oder Aminoalkoholate zugesetzt werden. Derartige weitere Zusatzstoffe sind in der EP-A 0 389 041, der EP-A 0 200 260 und der WO 95/19222 beschrieben, die diesbezüglich vollumfänglich in den Kontext der vorliegenden Anmeldung durch Bezugnahme einbezogen werden.

Statt basischer Zusatzstoffe ist es auch möglich, saure Zusatzstoffe zu verwenden. Bevorzugt sind organische saure Verbindungen, die sich nach dem Verformungsschritt durch Calcinieren herausbrennen lassen. Besonders bevorzugt sind Carbonsäuren.

Die Menge an diesen Hilfsstoffen beträgt vorzugsweise 1 bis 10 Gew.-%, besonders bevorzugt 2 bis 7 Gew.-%, sind jeweils bezogen auf den letztlich hergestellten Formkörper, wie untenstehend beschrieben.

Zur Beeinflussung von Eigenschaften des Formkörpers wie z.B. Transportporenvolumen, Transportporendurchmesser und Transportporenverteilung kann man weitere Substanzen, vorzugsweise organische Verbindungen, insbesondere organische Polymere als weitere Zusatzstoffe zugeben, die auch die Verformbarkeit der Masse beeinflussen können. Solche Zusatzstoffe sind unter anderem Alginate, Polyvinylpyrolidone, Stärke, Cellulose, Polyether, Polyester, Polyamide, Polyamine, Polyimine, Polyalkene, Polystyrol, Styrol-Copolymere, Polyacrylate, Polymethylacrylate, Fettsäuren wie beispielsweise Stearinsäure, hochmolekulare Polyalkylenglykole wie beispielsweise Polyethylenglykol, Polypropylenglykol oder Polybutylenglykol, oder Gemische aus zwei oder mehr davon eingesetzt werden. Die Gesamtmenge an diesen Stoffen, bezogen auf den letztlich hergestellten Formkörper, wie untenstehend beschrieben, beträgt vorzugsweise 0,5 bis 10 Gew.-%, besonders bevorzugt 1 bis 6 Gew.-%.

Demgemäß betrifft die vorliegende Erfindung auch die Verwendung von Polyalkylenglycol, insbesondere von Polyethylenglykol bei der Herstellung von titansilikalithaltigen Formkörpern, insbesondere solche, die als Katalysatoren für Selektivoxidation eingesetzt werden.
In einer bevorzugten Ausführungsform werden im erfindungsgemäßen Verfahren Formkörper hergestellt, die im wesentlichen mikroporös sind, darüberhinaus aber auch Meso- und/oder Makroporen aufweisen können. Das Porenvolumen der Meso- und Makroporen im erfindungsgemäßen Formkörper, bestimmt nach DIN 66133 durch Quecksilberporosimetrie, ist größer als 0,1 ml/g, bevorzugt größer als 0,2 ml/g, besonders bevorzugt größer als 0,3 ml/g, insbesondere größer als 0,5 ml/g.

Die Zugabereihenfolge der oben beschriebenen Zusatzstoffe zu dem Gemisch, das gemäß einem der oben beschriebenen Verfahren erhalten wurde, ist unkritisch. Es ist sowohl möglich, zuerst weiteres Metalloxid via Metalloxidsol, anschließend die viskositätssteigernden und dann die die Transporteigenschaften und/oder die Verformbarkeit der verdichteten Masse beeinflussenden Substanzen einzubringen, als auch jede beliebige andere Reihenfolge.
Wahlweise kann vor der Verdichtung das in der Regel noch pulverförmige Gemisch 10 bis 180 min im Kneter oder Extruder homogenisiert werden. Dabei wird in der Regel bei Temperaturen im Bereich von ungefähr 10 °C bis zum Siedepunkt des Anteigungsmittels und Normaldruck oder leichtem überatmosphärischem Druck gearbeitet. Das Gemisch wird solange geknetet, bis eine verstrangbare oder extrudierfähige Masse entstanden ist.

Die nach dem Verdichten zur Verformung anstehende Masse besitzt im erfindungsgemäßen Verfahren einen Anteil an Metalloxid von mindestens 10 Gew.-%, bevorzugt mindestens 15 Gew.-%, besonders bevorzugt mindestens 20 Gew.-%, insbesondere mindestens 30 Gew.-%, bezogen auf die Gesamtmasse. Insbesondere bei der Verwendung von titanhaltigen mikroporösen Oxiden zeichnet sich die im erfindungsgemäßen Verfahren hergestellte Masse dadurch aus, daß sie zu keinen Problemen bei der anstehenden Verformung aufgrund thixotroper Eigenschaften führt.
Prinzipiell können für die Knetung und die Verformung alle herkömmlichen Knet- und Verformungsvorrichtungen bzw. Verfahren, wie sie zahlreich aus dem Stand der Technik bekannt und für die Herstellung von z.B. Katalysator-Formkörpem geeignet sind, verwendet werden.

Vorzugsweise werden Verfahren verwendet, bei denen die Verformung durch Extrusion in üblichen Extrudern, beispielsweise zu Strängen mit einem Durchmesser von üblicherweise ungefähr 1 bis ungefähr 10 mm, insbesondere ungefähr 1,5 bis ungefähr 5 mm erfolgt. Derartige Extrusionsvorrichtungen werden beispielsweise in Ullmanns "Enzyklopädie der Technischen Chemie", 4. Auflage, Bd. 2 (1972), S. 295 ff. beschrieben. Neben der Verwendung eines Extruders wird ebenfalls vorzugsweise eine Strangpresse verwendet. Im Falle einer großtechnischen Anwendung des Verfahrens wird besonders bevorzugt mit Extrudern gearbeitet.

Die Extrudate sind entweder Stränge oder Wabenkörper. Die Form der Waben ist beliebig. Es kann sich dabei beispielsweise um Rundstränge, Hohlstränge, oder sternförmige Stränge handeln. Auch der Durchmesser der Waben ist beliebig. Über die äußere Form sowie den Durchmesser entscheiden in der Regel die prozeßtechnischen Anforderungen, die durch das Verfahren, in dem der Formkörper eingesetzt werden soll, vorgegeben werden.

Vor, während oder nach dem Formgebungsschritt können auf das Material Edelmetalle in Form geeigneter Edelmetallkomponenten, beispielsweise in Form von wasserlöslichen Salzen aufgebracht werden. Vorzugsweise wird dieses Verfahren angewendet, um Oxidationskatalysatoren auf der Basis von Titan- oder Vanadiumsilikaten mit Zeolithstruktur herzustellen, wobei Katalysatoren erhältlich sind, die einen Gehalt von 0,01 bis 30 Gew.-% an einem oder mehreren Edelmetallen aus der Gruppe Ruthenium, Rhodium, Palladium, Osmium, Iridium, Platin, Rhenium, Gold und Silber aufweisen. Derartige Katalysatoren sind beispielsweise in der DE-A 196 23 609.6 beschrieben, die hiermit bzgl. der darin beschriebenen Katalysatoren voll umfänglich in den Kontext der vorliegenden Anmeldung durch Bezugnahme aufgenommen wird.
In vielen Fällen ist es jedoch am günstigsten, die Edelmetallkomponenten erst nach dem Formgebungsschritt auf die Formkörper aufzubringen, besonders dann, wenn eine Hochtemperaturbehandlung des edelmetallhaltigen Katalysators unerwünscht ist. Die Edelmetallkomponenten können insbesondere durch Ionenaustausch, Imprägnierung oder Aufsprühen auf den Formkörper gebracht werden. Das Aufbringen kann mittels organischer Lösungsmittel, wäßriger ammoniakalischer Lösungen oder überkritischer Phasen wie etwa Kohlendioxid erfolgen.

Durch den Einsatz dieser vorgenannten Methoden können durchaus verschiedenartige edelmetallhaltige Katalysatoren erzeugt werden. So kann durch Aufsprühen der Edelmetallösung auf die Formkörper eine Art Schalenkatalysator erzeugt werden. Die Dicke dieser edelmetallhaltigen Schale läßt sich durch Imprägnieren deutlich vergrößern, während beim Ionenaustausch die Katalysatorpartikel weitgehend gleichmäßig über den Formkörperquerschnitt hinweg mit Edelmetall belegt werden.

Nach Beendigung des Strangpressens oder des Extrudierens werden die erhaltenen Formkörper bei im allgemeinen 50 bis 250 °C, bevorzugt 80 bis 250 °C bei Drücken von im allgemeinen 0,01 bis 5 bar, bevorzugt 0,05 bis 1,5 bar im Laufe von ungefähr 1 bis 20 h getrocknet.

Die anschließende Calcinierung erfolgt bei Temperaturen von 250 bis 800 °C, bevorzugt 350 bis 600 °C, besonders bevorzugt 400 bis 500 °C. Der Druckbereich wird ähnlich dem der Trocknung gewählt. In der Regel wird in sauerstoffhaltiger Atmosphäre calciniert, wobei der Sauerstoffgehalt 0,1 bis 90 Vol.-%, bevorzugt 0,2 bis 22 Vol.-%, besonders bevorzugt 0,2 bis 10 Vol.-% beträgt.

Die vorliegende Erfindung betrifft somit auch ein Verfahren zur Herstellung von Formkörpern, wie oben beschrieben, das die folgenden Stufen (i) bis(v) umfaßt:
(i) Vermischen des mindestens einen porösen oxidischen Materials mit mindestens einem Metalloxidsol, das einen niedrigen Gehalt an Alkali- und Erdalkalimetallionen aufweist, und/oder mindestens einem Metalloxid, das einen niedrigen Gehalt an Alkali- und Erdalkalimetallionen aufweist;
(ii) Verdichten des Gemisches aus Stufe (i), optional unter Zusatz von Metalloxidsol;
(iii) Verformen der Masse aus Stufe (ii);
(iv) Trocknen der Formkörper aus Stufe (iii);
(v) Calcinieren der getrockneten Formkörper aus Stufe (iv).
Eine spezielle Ausführungsform der Erfindung besteht darin, der Suspension, die aus der weiter oben beschriebenen Stufe (b) entsteht, das Metalloxidsol zuzusetzen, die resultierende Suspension zu trocknen, vorzugsweise durch Sprühtrocknung, und das resultierende Pulver zu calcinieren. Das getrocknete und calcinierte Produkt kann dann gemäß Stufe (iii) weiter verarbeitet werden.

Selbstverständlich können die erhaltenen Stränge oder Extrudate konfektioniert werden. Sämtliche Verfahren zur Zerkleinerung sind dabei denkbar, beispielsweise durch Splittung oder Brechen der Formkörper, ebenso wie weitere chemische Behandlungen, wie beispielsweise oben beschrieben. Findet eine Zerkleinerung statt, wird dabei vorzugsweise Granulat oder Splitt mit einem Partikeldurchmesser von 0,1 bis 5 mm, insbesondere 0,5 bis 2 mm erzeugt.

Dieses Granulat oder dieser Splitt und auch auf anderem Wege erzeugte Formkörper enthalten praktisch keine feinkörnigeren Anteile als solche mit ungefähr 0,1 mm Mindestpartikeldurchmesser.

Die erfindungsgemäß hergestellten bzw. erfindungsgemäßen Formkörper können als Katalysatoren, insbesondere zur katalytischen Umwandlung, vor allem zur Oxidation organischer Moleküle eingesetzt werden. Als Umsetzungen sind unter anderen zu nennen:
die Epoxidation von Olefinen wie z.B. die Herstellung von Propenoxid aus Propen und H₂O₂ oder aus Propen und Gemischen, die H₂O₂ in situ liefern;

Hydroxylierungen wie z.B die Hydroxylierung mono-, bi- oder polycyclischer Aromaten zu mono-, di- oder höher substituierten Hydroxyaromaten, beispielsweise die Umsetzung von Phenol und H₂O₂ oder von Phenol und Gemischen, die H₂O₂ in situ liefern, zu Hydrochinon;
die Umwandlung von Alkanen zu Alkoholen, Aldehyden und Säuren;
die Oximbildung aus Ketonen unter Anwesenheit von H₂O₂ oder Gemischen, die H₂O₂ in situ liefern, und Ammoniak (Ammonoximierung), beispielsweise die Herstellung von Cyclohexanonoxim aus Cyclohexanon;
Isomerisierungsreaktionen wie z.B. die Umwandlung von Epoxiden zu Aldehyden;
sowie weitere in der Literatur mit derartigen Formkörpern, insbesondere Zeolith-Katalysatoren beschriebene Umsetzungen, wie sie beispielsweise von W. Hölderich in "Zeolites: Catalysts for the Synthesis of Organic Compounds", Elsevier, Stud. Surf. Sci. Catal., 49, Amsterdam (1989), S. 69 bis 93, und insbesondere für mögliche Oxidationsreaktionen von B. Notari in Stud. Surf. Sci. Catal., 37 (1987), S. 413 bis 425, oder in Advances in Catalysis, Vol. 41, Academic Press (1996), S. 253 bis 334 beschrieben sind.

Daher betrifft die vorliegende Erfindung die Verwendung eines wie oben beschrieben hergestellten Formkörpers oder eines Gemisches aus zwei oder mehr davon als Katalysator.

Dabei eignen sich die vorstehend ausführlich diskutierten Zeolithe insbesondere für die Epoxidation von Alkenen.
Demgemäß betrifft die vorliegende Erfindung auch ein Verfahren zur Herstellung mindestens eines Alkenoxids, das den folgenden Schritt (III) umfaßt:
(III) Umsetzung mindestens eines Alkens mit Wasserstoffperoxid an einem Katalysator,
dadurch gekennzeichnet, daß als Katalysator ein Formkörper, hergestellt in einem Verfahren, wie oben beschrieben, oder ein Formkörper, wie oben beschrieben, eingesetzt wird.

Alkene, die für eine solche Funktionalisierung durch Epxidation in Frage kommen, sind beispielsweise Ethen, Propen, 1-Buten, 2-Buten, Isobuten, Butadien, Pentene, Piperylen, Hexene, Hexadiene, Heptene, Octene, Diisobuten, Trimethylpenten, Nonene, Dodecen, Tridecen, Tetra- bis Eicosene, Tri- und Tetrapropen, Polybutadiene, Polyisobutene, Isoprene, Terpene, Geraniol, Linalool, Linalylacetat, Methylencyclopropan, Cyclopenten, Cyclohexen, Norbornen, Cyclohepten, Vinylcyclohexan, Vinyloxiran, Vinylcyclohexen, Styrol, Cycloocten, Cyclooctadien, Vinylnorbornen, Inden, Tetrahydroinden, Methylstyrol, Dicyclopentadien, Divinylbenzol, Cyclododecen, Cyclododecatrien, Stilben, Diphenylbutadien, Vitamin A, Betacarotin, Vinylidenfluorid, Allylhalogenide, Crotylchlorid, Methallylchlorid, Dichlorbuten, Allylalkohol, Methallylalkohol, Butenole, Butendiole, Cyclopentendiole, Pentenole, Octadienole, Tridecenole, ungesättigte Steroide, Ethoxyethen, Isoeugenol, Anethol, ungesättigte Carbonsäuren wie z.B. Acrylsäure, Methacrylsäure, Crotonsäure, Maleinsäure, Vinylessigsäure, ungesättigte Fettsäuren, wie z.B. Ölsäure, Linolsäure, Palmitinsäure, natürlich vorkommende Fette und Öle.

Vorzugsweise eignen sich die vorstehend ausführlich diskutieren Zeolithe für die Epoxidation von Alkenen mit 2 bis 8 C-Atomen, weiter bevorzugt von Ethen, Propen oder Buten, und insbesondere von Propen zu den entsprechenden Alkenoxiden.

Demgemäß betrifft die vorliegende Erfindung insbesondere die Verwendung des hierin beschriebenen Formkörpers als Katalysator zur Herstellung von Propenoxid, ausgehend von Propen und Wasserstoffperoxid oder von Propen und Gemischen, die H₂O₂ in situ liefern.

In einer speziellen Ausführungsform des Verfahrens wird das zu epoxidierende Alken durch Dehydrierung des entsprechenden Alkans hergestellt.

Demzufolge betrifft die vorliegende Erfindung auch ein Verfahren, wie oben beschrieben, das den zusätzlichen Schritt (I) umfaßt:
(I) Herstellung des in Schritt (III) umgesetzten, mindestens einen Alkens durch Dehydrierung mindestens eines Alkans.

Diese Dehydrierung kann prinzipiell gemäß allen, aus dem Stand der Technik bekannten Verfahren durchgeführt werden. Solche Verfahren sind unter anderem in der EP-A 0 850 936 beschrieben, die diesbezüglich vollumfänglich in den Kontext dieser Anmeldung durch Bezugnahme aufgenommen wird.

In einer bevorzugten Ausführungsform des erfindungsgemäßen Verfahrens wird der Wasserstoff, der bei der Dehydrierung des mindestens einen Alkans erzeugt wird, dazu verwendet, Wasserstoffperoxid herzustellen, mit dem das bei der Dehydrierung erzeugte, mindestens eine Alken in Schritt (III) umgesetzt wird.

Demgemäß betrifft die vorliegende Erfindung auch ein Verfahren, wie oben beschrieben, das den folgenden Schritt (II) umfaßt:
(II) Umsetzung des in Schritt (I) entstehenden Wasserstoffs zu Wasserstoffperoxid,
wobei das Wasserstoffperoxid zur Umsetzung in Schritt (III) eingesetzt wird.

Folglich betrifft die vorliegende Erfindung auch ein integriertes Verfahren zur Herstellung eines Alkenoxids, das die Schritte (A) bis (C) umfaßt:
(A) Dehydrierung eines Alkans zu einem Alken unter Erhalt von Wasserstoff,
(B) Umsetzung des in (A) erhaltenen Wasserstoffs zu Wasserstoffperoxid,
(C) Umsetzung des Wasserstoffperoxids aus (B) mit dem Alken aus (A) unter Erhalt des Alkenoxids, unter Verwendung eines erfindungsgemäßen Formkörpers.

Die Umsetzung des Wasserstoffs zu Wasserstoffperoxid kann hierbei nach allen Verfahren, die aus dem Stand der Technik bekannt sind, durchgeführt werden. Insbesondere kann der Wasserstoff mit molekularem Sauerstoff zu Wasserstoffperoxid umgesetzt werden. Ebenso ist es denkbar, unter Verwendung des Wasserstoffes aus Schritt (A) über das Anthrachinonverfahren Wasserstoffperoxid herzustellen. In beiden Fällen kann es hierbei erforderlich sein, den Wasserstoff aus Schritt (A) vor der weiteren Verwendung zu reinigen. Bevorzugt wird jedoch auf das Anthrachinonverfahren zurückgegriffen. Dieses beruht auf der katalytischen Hydrierung einer Anthrachinon-Verbindung zur entsprechenden Anthrahydrochinon-Verbindung, nachfolgender Umsetzung derselben mit Sauerstoff unter Bildung von Wasserstoffperoxid und anschließender Abtrennung des gebildeten Wasserstoffperoxids durch Extraktion. Der Katalysezyklus wird durch erneute Hydrierung der rückgebildeten Anthrachinon-Verbindung geschlossen. Einen Überblick über das Anthrachinonverfahren gibt "Ullmanns Encyclopedia of Industrial Chemistry", 5. Auflage, Band 13, Seiten 447 bis 456.

Bei Verwendung eines erfindungsgemäß hergestellten Formkörpers oder mehr davon als Katalysator kann dieser nach erfolgter Deaktivierung durch ein Verfahren regeneriert werden, bei dem die Regenerierung durch gezieltes Abbrennen der für die Deaktivierung verantwortlichen Beläge erfolgt. Dabei wird bevorzugt in einer Inertgasatmosphäre gearbeitet werden, die genau definierte Mengen an Sauerstoff-liefernden Substanzen enthält. Dieses Regenerierungsverfahren ist in der DE-A 197 23 949.8 beschrieben, die diesbezüglich vollumfänglich in den Kontext der vorliegenden Anmeldung durch Bezugnahme einbezogen werden.

Darüberhinaus betrifft die vorliegende Erfindung in ihrer allgemeinsten Ausgestaltungsform die Verwendung eines Metalloxidsols, hergestellt wie oben beschrieben, als Bindemittel zur Herstellung eines Formkörpers hoher chemischer und mechanischer Festigkeit.

Die nachfolgenden Beispiele sollen die Erfindung erläutern.

### Beispiele

### Beispiel 1: Herstellung eines mikroporösen oxidischen Materials

In einem Vierhalskolben (41 Inhalt) wurden 910 g Tetraethylorthosilicat vorgelegt und aus einem Tropftrichter innerhalb von 30 min mit 15 g Tetraisopropylorthotitanat unter Rühren (250 U/min, Blattrührer) versetzt. Es bildete sich eine farblose, klare Mischung. Anschließend versetzte man mit 1600 g einer 20 gew.-%igen Tetrapropylammoniumhydroxid-Lösung (Alkalimetallgehalt < 10 ppm) und rührte noch 1 h nach. Bei 90 bis 100 °C wurde das aus der Hydrolyse gebildete Alkoholgemisch (ca. 900 g) abdestilliert. Man füllte mit 3 1 Wasser auf und gab das mittlerweile leicht opaque Sol in einen 5 1 fassenden Rührautoklaven aus Edelstahl.

Mit einer Heizrate von 3 °C/min wurde der verschlossene Autoklav (Ankerrührer, 200 U/min) auf eine Reaktionstemperatur von 175 °C gebracht. Nach 92 h war die Reaktion beendet. Das erkaltete Reaktionsgemisch (weiße Suspension) wurde abzentrifugiert und mehrfach mit Wasser neutral gewaschen. Der erhaltene Feststoff wurde bei 110 °C innerhalb von 24 h getrocknet (Auswaage: 298 g).
Anschließend wurde unter Luft bei 550 °C in 5 h das im Zeolithen verbliebene Templat abgebrannt (Calcinierungsverlust: 14 Gew.-%).
Das reinweiße Produkt hatte nach naßchemischer Analyse einen Ti-Gehalt von 1,5 Gew.-% und einen Restgehalt an Alkalimetall unterhalb 100 ppm. Die Ausbeute auf eingesetztes Siliciumdioxid betrug 97 %. Die Kristallite hatten eine Größe von 0,05 bis 0,25 µm und das Produkt zeigte im IR eine typische Bande bei ca. 960 cm⁻¹.

### Beispiel 2: Herstellung eines Kieselsols

3 l Wasser wurden in einem 10 l Vierhalskolben, versehen mit Rührer, Thermometer und Rückflußkühler, vorgelegt. Mit 6 g 25 %igem Ammoniak wurde der pH-Wert der Lösung auf 8 bis 9 eingestellt. Anschließend wurden das Wasser auf 50 °C erwärmt und 1300 g Tetraethylorthosilicat mit Hilfe eines Tropftrichters zugetropft.

Die Mischung aus Wasser und Tetraethylorthosilicat wurde 3 h unter Rückfluß gekocht. Anschließend gab man weitere 1304 g Tetraethylorthosilicat über einen Tropftrichter zu. Nach weiteren 2 h Kochen unter Rückfluß ließ man die so erhaltene Kieselsol-Wassermischung weitere 12 h rühren und destillierte dann das durch die Hydrolyse entstandene Ethanol ab.

Die so erzeugten 3618 g Kieselsol hatten einen Siliciumdioxidgehalt von ca. 20 Gew.-% und einen Gehalt an Alkalimetallionen kleiner 3 ppm.

### Beispiel 3: Herstellung eines Kieselsols

188,6 g Wasser wurden in einem 500 ml Vierhalskolben, versehen mit Rührer, Thermometer und Rückflußkühler, vorgelegt. Mit 0,3 g 25 %igem Ammoniak wurde der pH-Wert der Lösung auf 9 eingestellt. Anschließend wurden das Wasser auf 50 °C erwärmt und 111,65 g Tetraethylorthosilicat mit Hilfe eines Tropftrichters zugetropft.

Die Mischung aus Wasser und Tetraethylorthosilicat wurde 2 h unter Rückfluß gekocht. Anschließend gab man weitere 111,65 g Tetraethylorthosilicat über einen Tropftrichter zu. Nach weiteren 2 h Kochen unter Rückfluß ließ man die so erhaltene Kieselsol-Wasser-Mischung weitere 12 h rühren. Anschließend gab man 50 g Wasser zu und destillierte dann das durch die Hydrolyse entstandene Ethanol ab.

Die so erzeugten 169 g Kieselsol hatten einen Siliciumdioxidgehalt von ca. 38 Gew.-% und einen Gehalt an Alkalimetallionen kleiner 5 ppm.

### Beispiel 4: Versprühen von Titansilicalit

200 g gemahlener Katalysator, hergestellt gemäß Beispiel 1, wurden zunächst auf eine Partikelgröße von < 300 µm feingemahlen und dann in 2000 g Wasser suspendiert. Anschließend wurden 245 g wäßriges Kieselsol mit einem Siliciumdioxidgehalt von 18 Gew.-%, hergestellt nach Beispiel 2, beigemischt.

Die Suspension wurde unter ständigem Rühren mit einer Schlauchpumpe zu einem Laborsprühturm aus Glas (Durchmesser: 200 mm, Höhe des zylindrischen Teils: 500 mm) gefördert und mittels einer Zweistoffdüse (Durchmesser der Flüssigkeitszuleitung: 2,5 mm, Düsengas-Vordruck: 3 bar) zerstäubt.
Im Sprühturm wurde die Suspension durch das Trockengas (Stickstoff, Durchsatz: 24 kg/h, Eintrittstemperatur: 210 °C, Austrittstemperatur: 100 °C) zu einem feinen, innig durchmischten Pulver getrocknet, das dann in einem Glaszyklon abgeschieden wurde. Die Ausbeute betrug 80 %.

### Beispiel 5: Versprühen von Titansilicalit

16,1 kg Katalysator, hergestellt gemäß Beispiel 1, wurden zunächst mit einer Hammermühle grob vorgemahlen und danach mit einer Pralltellermühle auf eine Partikelgröße von < 300 µm feingemahlen.
Das Pulver wurde anschließend unter Zugabe von 16 kg wäßrigen Kieselsols mit einem Siliciumdioxidgehalt von 20 Gew.-%,, hergestellt gemäß Beispiel 2, in 160 1 Wasser suspendiert und in einen offenen Rührkessel gefüllt. Aus diesem Behälter wurde die Suspension unter ständigem Rühren mit Hilfe einer großen Schlauchpumpe abgezogen und in einer Sprühtrocknungsanlage (der Firma Niro) zu einem feinen, innig durchmischten Pilver getrocknet.

Zur Dispergierung der Suspension wurde eine Zerstäuberscheibe mit Keramikhülsen (Drehzahl 17000 U/min) eingesetzt. Die Trocknung erfolgte bei einer Luft-Eintrittstemperatur von 260 °C und einer Luft-Austrittstemperatur von 110°C

Das Produkt wurde vom Luftstrom in einem Zyklon abgetrennt. Die Ausbeute betrug 13 kg.

### Vergleichsbeispiel 1: Verformung von Titansilicalit (Katalysator A)

Der Katalysator A wurde hergestellt durch Mischen von 1665 g eines Sprühpulvers, bestehend aus 89 Gew.-% eines Katalysators, hergestellt gemäß Beispiel 1, und 11 Gew.-% Siliciumdioxid, mit 416 g eines Kieselsols eines Siliciumdioxidgehalts von ca. 50 Gew.-% (Ludox-TM, der Firma DuPont).
Das genannte Sprühpulver wurde hergestellt wie das in Beispiel 4 beschriebene, nur daß statt des erfindungsgemäß hergestellten Kieselsols ein kommerziell hergestelltes Kieselsol (Ludox AS-40 der Firma DuPont) mit einem Natriumgehalt von 800 ppm verwendet wurde.

Die Mischung wurde durch Zugabe von Wasser und dem Verstrangungshilfsmittel Methylcellulose extrusionsfähig gemacht und zu Strängen von 1,5 mm Durchmesser extrudiert.

Diese Stränge wurden bei 120 °C getrocknet und 5 h bei 500 °C getempert. Der Gehalt an Siliciumdioxid-Binder im Formkörper betrug 20 Gew.-%, der Natriumgehalt 700 ppm.

### Vergleichsbeispiel 2: Verformung von Titansilicalit (Katalysator B)

Der Katalysator B wurde hergestellt durch Mischen von 3000 g eines Sprühpulvers, bestehend aus 78 Gew.-% eines Katalysators, hergestellt gemäß Beispiel 1, und 22 Gew.-% Siliciumdioxid, mit 750 g eines Kieselsols eines Siliciumdioxidgehalts von ca. 43 Gew.-% (Ludox AS-40 der Firma DuPont).

Das genannte Sprühpulver wurde hergestellt wie das in Beispiel 4 beschriebene, nur daß statt des erfindungsgemäß hergestellten Kieselsols ein kommerziell hergestelltes Kieselsol (Ludox AS-40 der Firma DuPont) mit einem Natriumgehalt von 800 ppm verwendet wurde.

Die Mischung wurde durch Zugabe von Wasser und dem Verstrangungshilfsmittel Methylcellulose extrusionsfähig gemacht und zu Strängen von 2,5 mm Durchmesser extrudiert.

Diese Stränge wurden bei 120 °C getrocknet und 5 h bei 500 °C getempert. Der Gehalt an Siliciumdioxid-Binder im Formkörper betrug 30 Gew.-%, der Natriumgehalt 910 ppm. Die Seitendruckfestigkeit der Stränge betrug 37,9 N, die Schnitthärte 10,25 N.

### Vergleichsbeispiel 3: Verformung von Titansilicalit (Katalysator C)

Der Katalysator C wurde hergestellt durch Kollern von 7500 g eines Sprühpulvers, bestehend aus 78 Gew.-% eines Katalysators, hergestellt gemäß Beispiel 1, und 22 Gew.-% Siliciumdioxid, mit 4300 g eines Kieselsols eines Siliciumdioxidgehalts von ca. 43 Gew.-% (Ludox AS-40 der Firma DuPont).
Das genannte Sprühpulver wurde hergestellt wie das in Beispiel 4 beschriebene, nur daß statt des erfindungsgemäß hergestellten Kieselsols ein kommerziell hergestelltes Kieselsol (Ludox AS-40 der Firma DuPont) mit einem Natriumgehalt von 800 ppm verwendet wurde.

Die Mischung wurde durch Zugabe von Wasser und dem Verstrangungshilfsmittel Methylcellulose extrusionsfähig gemacht und zu Strängen von 1,5 mm Durchmesser extrudiert.

Diese Stränge wurden bei 120 °C getrocknet und 5 h bei 500 °C getempert. Der Gehalt an Siliciumdioxid-Binder im Formkörper betrug 30 Gew.-%, der Natriumgehalt 900 ppm.

### Beispiel 6: Verformung von Titansilicalit: (Katalysator D)

Der Katalysator D wurde hergestellt durch Mischen von 2200 g eines Sprühpulvers, bestehend aus 75 Gew.-% eines Katalysators, hergestellt gemäß Beispiel 1, und 25 Gew.-% Siliciumdioxid, mit 1037 g eines Kieselsols mit einem Siliciumdioxidgehalt von ca. 21 Gew.-%, hergestellt gemäß Beispiel 2.
Das genannte Sprühpulver wurde hergestellt analog Beispiel 4.

Die Mischung wurde durch Zugabe von Wasser und dem Verstrangungshilfsmittel Methylcellulose extrusionsfähig gemacht und zu Strängen von 1,5 mm Durchmesser extrudiert.

Diese Stränge wurden bei 120 °C getrocknet und 5 h bei 500 °C getempert. Der Gehalt an Siliciumdioxid-Binder im Formkörper betrug 32 Gew.-%, der Natriumgehalt 400 ppm.

### Beispiel 7: Verformung von Titansilicalit: (Katalysator E)

Der Katalysator E wurde hergestellt durch Kollern von 9700 g eines Sprühpulvers, bestehend aus 75 Gew.-% eines Katalysators, hergestellt gemäß Beispiel 1, und 25 Gew.-% Siliciumdioxid, mit 13000 g eines Kieselsols mit einem Siliciumdioxidgehalt von ca. 19 Gew.-%, hergestellt gemäß Beispiel 2.

Das genannte Sprühpulver wurde hergestellt analog Beispiel 4.

Die Mischung wurde durch Zugabe von Wasser und dem Verstrangungshilfsmittel Methylcellulose extrusionsfähig gemacht und zu Strängen von 1,5 mm Durchmesser strangverpreßt.

Diese Stränge wurden bei 120 °C getrocknet und 5 h bei 500 °C getempert. Der Gehalt an Siliciumdioxid-Binder im Formkörper betrug 40 Gew.-%, der Natriumgehalt 420 ppm.

### Beispiel 8: Verformung von Titansilicalit: (Katalysator F)

Der Katalysator F wurde hergestellt durch Kollern von 8000 g eines Sprühpulvers, bestehend aus 70 Gew.-% eines Katalysators, hergestellt gemäß Beispiel 1, und 30 Gew.-% Siliciumdioxid, mit 4000 g eines Kieselsols mit einem Siliciumdioxidgehalt von ca. 19 Gew.-%, hergestellt gemäß Beispiel 2.

Das genannte Sprühpulver wurde hergestellt analog Beispiel 4.

Die Mischung wurde durch Zugabe von Wasser und dem Verstrangungshilfsmittel Methylcellulose extrusionsfähig gemacht und zu Strängen von 1,5 mm Durchmesser extrudiert.

Diese Stränge wurden bei 120 °C getrocknet und 5 h bei 500 °C getempert. Der Gehalt an Siliciumdioxid-Binder im Formkörper betrug 40 Gew.-%, der Natriumgehalt 400 ppm. Die Schnitthärte betrug 2 N und die Seitendruckfestigkeit 19 N.

### Beispiel 9: Verdichtung und Verformung von Titansilicalit (Katalysator G)

3,5 kg TS-1, hergestellt gemäß Beispiel 1, wurden in einem Koller mit 1,23 kg Aerosil® (DEGUSSA), 6,26 kg Kieselsol, hergestellt gemäß Beispiel 2, und 237 g Methylcellulose (Walocel®) in einer Zeit von 60 min verdichtet.

Anschließend wurden 48 g Polyethylenglykol (ALKOX-E160®) zugesetzt, die Mischung weitere 30 min verdichtet, 96 g Polyethylenglykol (ALKOX-E160®) und 450 g VE-Wasser zugesetzt und die Mischung noch einmal 15 min verdichtet.

Die verformbare Masse wurde mit Hilfe eines Extruders zu 1,5 mm-Rundsträngen verformt. Dabei betrug der Auspreßdruck 85 bis 100 bar, die Auspreßzeit 15 min. Diese Stränge wurden bei 120 °C getrocknet und bei 500 °C in Luft 5 h calciniert.

Die Ausbeute betrug 5,1 kg. Der Gehalt an Siliciumdioxid-Binder im Formkörper betrug 40 Gew.-%, der Natriumgehalt 500 ppm, die Seitendruckfestigkeit 17 N und das Makroporenvolumen 0,70 g/ml, bestimmt durch Hg-Porosimetrie nach DIN 66133.

### Beispiel 10: Katalytische Testreihe (Batchbetrieb)

In einen Stahlautoklaven mit Korbeinsatz und Begasungsrührer wurden jeweils soviel Gramm an Katalysator A bis G eingebaut, daß die Masse an eingebautem Titansilicalit jeweils 0,5 g beträgt.

Der Autoklav wurde mit 100 g Methanol befüllt, verschlossen und auf seine Dichtigkeit überprüft. Anschließend wurden der Autoklav auf 40 °C temperiert und 11 g flüssiges Propen in den Autoklaven dosiert.
Nun wurden mittels einer HPLC-Pumpe 9,0 g einer 30 gew.-%igen wäßrigen Wasserstoffperoxidlösung in den Autoklaven gepumpt und die Wasserstoffperoxidreste in den Zuleitungen anschließend mit 16 ml Methanol in den Autoklaven gespült. Der Anfangsgehalt der Reaktionslösung an Wasserstoffperoxid betrug 2,5 Gew.-%.

Nach 2 h Reaktionszeit wurde der Autoklav abgekühlt und entspannt. Der flüssige Austrag wurde cerimetrisch auf Wasserstoffperoxid untersucht. Die Analyse und die Bestimmung des Gehalts des Austrags an Propylenoxid erfolgte gaschromatographisch.

In der nachfolgenden Tabelle sind die Ergebnisse der Analyse zusammengefaßt.

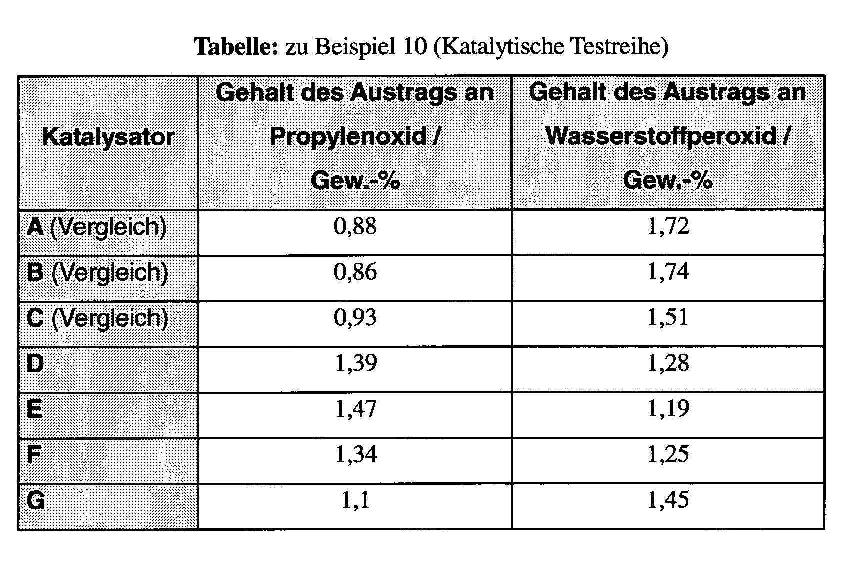

### Beispiel 11: Katalytischer Test (Kontinuierlicher Betrieb)

Durch einen Rohrreaktor, gefüllt mit 28,1 g des erfindungsgemäßen Katalysators F wurden Flüsse von 24 g Wasserstoffperoxid (40 Gew.-%) / h, 57 g Methanol / h und 11,7 ml Propen / h bei einer Reaktionstemperatur von 40 °C und einem Druck von 20 bar geleitet.

Nach Verlassen des Reaktors wurde die Reaktionsmischung in einem Sambay-Verdampfer gegen Atmosphärendruck entspannt. Die abgetrennten Leichtsieder wurden online in einem Gaschromatographen analysiert. Der flüssige Reaktionsaustrag wurde gesammelt, gewogen und ebenfalls gaschromatographisch analysiert.

Die gesamte Reaktionsdauer betrug 550 h. Während dieser Zeit lag der Wasserstoffperoxid-Umsatz bei weit über 90 %. Die Selektivität von Wasserstoffperoxid zu Propylenoxid lag im gesamten Zeitraum ebenfalls bei wesentlich mehr als 90 %.

### Beispiel 12: Katalytischer Test (Kontinuierlicher Betrieb)

Durch einen Rohrreaktor, gefüllt mit 20 g des erfindungsgemäßen Katalysators G, wurden Flüsse von 9 g Wasserstoffperoxid (40 Gew.-%) / h, 49 g Methanol / h und 8 g Propen / h bei einer Reaktionstemperatur von 40 °C und einem Druck von 20 bar geleitet.

Nach Verlassen des Reaktors wurde die Reaktionsmischung in einem Sambay-Verdampfer gegen Atmosphärendruck entspannt. Die abgetrennten Leichtsieder wurden online in einem Gaschromatographen analysiert. Der flüssige Reaktionsaustrag wurde gesammelt, gewogen und ebenfalls gaschromatographisch analysiert.

Die gesamte Reaktionsdauer betrug 850 h. Während dieser Zeit lag der Wasserstoffperoxid-Umsatz bei weit über 90 %. Die Selektivität von Wasserstoffperoxid zu Propylenoxid lag im gesamten Zeitraum ebenfalls bei wesentlich mehr als 90 %.

## Patentansprüche

1. Verfahren zur Herstellung eines Formkörpers, umfassend mindestens ein poröses oxidisches Material und mindestens ein Metalloxid, das die folgenden Stufen (i) bis (iii) umfaßt:
(i) Vermischen des mindestens einen porösen oxidischen Materials mit mindestens einem Metalloxidsol, das einen niedrigen Gehalt an Alkali- und Erdalkalimetallionen aufweist, und/oder mindestens einem Metalloxid, das einen niedrigen Gehalt an Alkali- und Erdalkalimetallionen aufweist, durch Versprühen einer Suspension, enthaltend das mindestens eine poröse oxidische Material und Metalloxidsol und/oder Metalloxid,
(ii) Verdichten des Gemisches aus Stufe (i) unter Zusatz von Metalloxidsol,
(iii) Verformen der Masse aus Stufe (ii),
wobei das in (i) oder (i) und (ii) eingesetzte Metalloxidsol einen Gehalt an Alkali- und Erdalkalimetallionen von weniger als 10 ppm und das in
(i) eingesetzte Metalloxid einen Gehalt an Alkali- und Erdalkalimetallionen von weniger als 200 ppm aufweisen.

2. Verfahren nach Anspruch 1, **dadurch gekennzeichnet, dass** es die Stufen (i) bis (iii) umfaßt:
(i) Vermischen des mindestens einen porösen oxidischen Materials mit mindestens einem Metalloxidsol, das einen niedrigen Gehalt an Alkali- und Erdalkalimetallionen aufweist, durch Versprühen einer Suspension, enthaltend das mindestens eine poröse oxidische Material und Metalloxidsol,
(ii) Verdichten des Gemisches aus Stufe (i), wobei weiteres Metalloxid eingebracht wird und als Metalloxidquelle Metalloxidsol dient,
(iii) Verformen der Masse aus Stufe (ii),
wobei das in (i) und (ii) eingesetzte Metalloxidsol einen Gehalt an Alkali- und Erdalkalimetallionen von weniger als 10 ppm aufweist.

3. Verfahren nach Anspruch 1 oder 2, **dadurch gekennzeichnet, daß** das Metalloxidsol hergestellt wird durch Hydrolyse eines Metallsäureesters.

4. Verfahren nach Anspruch 3, **dadurch gekennzeichnet, daß** der Metallsäureester ein Orthokieselsäureester ist.

5. Verfahren nach Anspruch 3 oder 4, **dadurch gekennzeichnet, dass** die Hydrolyse des Metallsäureesters bei einem pH-Wert von 7 bis 9 erfolgt.

6. Verfahren nach einem der Ansprüche 1 bis 5, **dadurch gekennzeichnet, daß** das poröse oxidische Material ein Zeolith ist.

7. Verfahren nach Anspruch 6, **dadurch gekennzeichnet, dass** der Zeolith ein Titansilikalit ist.

8. Verfahren nach einem der Ansprüche 1 bis 7, umfassend die Stufen (iv) und (v):
(iv) Trocknen der Formkörper aus Stufe (iii),
(v) Calcinieren der getrockneten Formkörper aus Stufe (iv).

9. Verfahren nach Anspruch 8, **dadurch gekennzeichnet, dass** die Trocknung gemäß Stufe (iv) bei 50 bis 250 °C und Drücken von 0,01 bis 5 bar im Laufe von 1 bis 20 h und die Calcinierung gemäß Stufe (v) bei Temperaturen von 250 bis 800 °C bei Drücken von 0,01 bis 5 bar erfolgt.

10. Verfahren nach einem der Ansprüche 1 bis 9, **dadurch gekennzeichnet, dass** die nach dem Verdichten gemäß (ii) zur Verformung anstehende Masse einen Anteil an Metalloxid von mindestens 20 Gew.-%, bezogen auf die Gesamtmasse, aufweist.

11. Formkörper mit einem Gehalt an Alklai- und Erdalkalimetallionen von weniger als 500 ppm, umfassend mindestens ein poröses oxidisches Material und mindestens ein Metalloxid, herstellbar durch ein Verfahren gemäß einem der Ansprüche 1 bis 10.

12. Formkörper nach Anspruch 11, umfassend Mikro-, Meso- und Makroporen, wobei das Porenvolumen der Meso- und Makroporen, bestimmt nach DIN 66133, größer als 0,1 ml/g ist.

13. Formkörper nach Anspruch 11 oder 12, **dadurch gekennzeichnet, dass** das poröse oxidische Material ein Zeolith, bevorzugt ein Titansilikalit, das Metalloxid Siliciumdioxid ist und das Metalloxidsol Kieselsol ist.

14. Verwendung eines Formkörpers, erhältlich durch ein Verfahren gemäß einem der Ansprüche 1 bis 10 oder eines Formkörpers gemäß einem der Ansprüche 11 bis 13 oder eines Gemisches aus zwei oder mehr davon als Katalysator.

15. Verwendung nach Anspruch 14, **dadurch gekennzeichnet, dass** der Formkörper als Katalysator zur Herstellung von Propenoxid, ausgehend von Propen und Wasserstoffperoxid eingesetzt wird.
